# EUROPEAN PATENT APPLICATION

(11) **EP 2 610 251 A1**
(43) Date of publication of application: **03.07.2013**
(21) Application number: 11460067.9
(22) Date of filing: 29.12.2011
(51) Int. Cl.: C07D 401/12, A61K 31/4402, A61P 7/02

(54) **Novel polymorphic forms of dabigatran etexilate and process for the preparation thereof**

(71) Applicant: Zaklady Farmaceutyczne Polpharma SA, 83-200 Starogard Gdanski (PL)
(72) Inventor: Zyla, Daniel, Starogard Gdanski (PL); Ignatowicz, Dawid, 80-299 Gdansk (PL); Szulc, Marcin, 83-200 Starogard Gdanski (PL)
(74) Representative: Tar, Miklos

(57) **Abstract**

The present invention relates to novel ethyl 3-[N-(2-{(4-(hexyloxycarbonylamino-iminomethyl)-phenylamino]-methyl}-1-methyl-1H-benzimidazole-5-carbonyl)-N-pyridin-2-yl-amino]-propanonate crystalline forms, process for manufacturing and use thereof.

## Description

### Field of the invention

The present invention relates to a novel polymorphic forms of ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate. The invention further relates to process for the preparation of such forms, to pharmaceutical formulations comprising the compound in such forms and to the therapeutic use of such forms.

### Background of the Invention

It is well-known that in the formulation of drug compositions it is important for the drug substance to be in a form in which it can be conveniently handled and processed. This is of importance, not only from the point of view of obtaining a commercially viable manufacturing process, but also from the point of subsequent manufacture of pharmaceutical formulations comprising the active compound. Chemical stability, solid state stability, and shelf life of the active ingredients are also very important factors. The drug substance, and the compositions containing it, should be capable of being effectively stored over appreciable periods of time, without exhibiting a significant change in the active component's physico-chemical characteristics.

Moreover, it is also important to be able to provide active pharmaceutical ingredients (APIs) in a form which is as pure as possible. The requirements set forth by the International Conference on Harmonization (ICH) with respect to APIs allowed to be administered to humans require that new and more efficient methods for the reduction of impurities in active substances should be sought. APIs in solid formulations may be in amorphous or crystalline forms. Amorphous materials may present significant problems regarding the purity. For example, such materials are typically more difficult to handle and to formulate than crystalline material, provide for unreliable solubility, and are often found to be unstable and chemically impure. The skilled person will appreciate that, if a drug can be readily obtained in a stable crystalline form, the above problems may be solved. Thus, in the manufacture of commercially viable and pharmaceutically acceptable, drug compositions, it is desirable, wherever possible, to provide drug in a substantially crystalline, and stable, form. It is to be noted, however, that this goal is not always achievable. Indeed, typically, it is not possible to predict, from molecular structure alone, what the crystallization behavior of a compound will be, and this can usually only be determined empirically.

Ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate 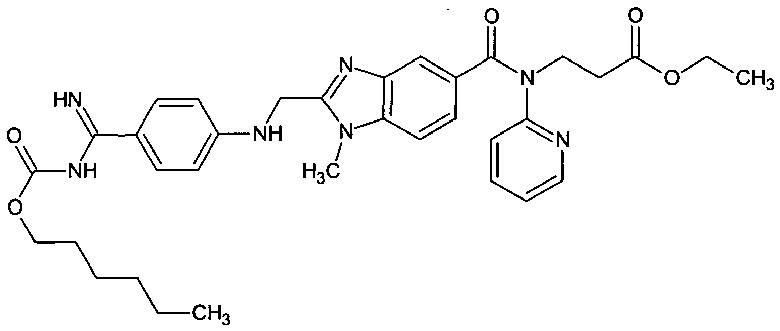 was first described in the PCT application No. WO98/37075 and is known under the common name dabigatran etexilate. Dabigatran etexilate is an orally active prodrug of the thrombin (Factor IIa) inhibitor dabigatran and is marketed under trade name Pradaxa.

The preparation of ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate was described in the Example 113 of WO98/37075. According to this example the obtained raw product was purified by column chromatography which cannot be applied economically and simply in industrial scale.

In the PCT application No. WO2006/131491 three different polymorphs of dabigatran etexilate were described namely anhydrous form (I), anhydrous form (II) and tetrahydrate form.

In the PCT application No. WO2008/059029 further five different polymorphs of dabigatran etexilate were described, namely anhydrous form (III), anhydrous form (IV), monohydrate (I), monohydrate (II) and solvate (I); a solvate with nitrobenzene. The described processes of preparation were laboratory processes and not adaptable in industrial scale as well.

It was obvious from the prior art that the known processes were not applicable in industrial scale or were not adequate to obtain products with the appropriate high purity. Thus there was a need for an economical and simple process which are applicable in industrial scale.

It was surprisingly been found that the mentioned problems are solved by the new purification process and the novel polymorphic forms of ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate of the present invention.

### Brief description of the Invention

The present invention relates to novel polymorphic forms of ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate known as form P and form Q. The invention further relates to process for the preparation of such forms by crystallization from isopropanol and to pharmaceutical formulations comprising the compound in such forms and to the therapeutic use of such forms.

### Detailed description of the Invention

The present invention relates to a novel polymorphic forms of ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate, form P and form Q.

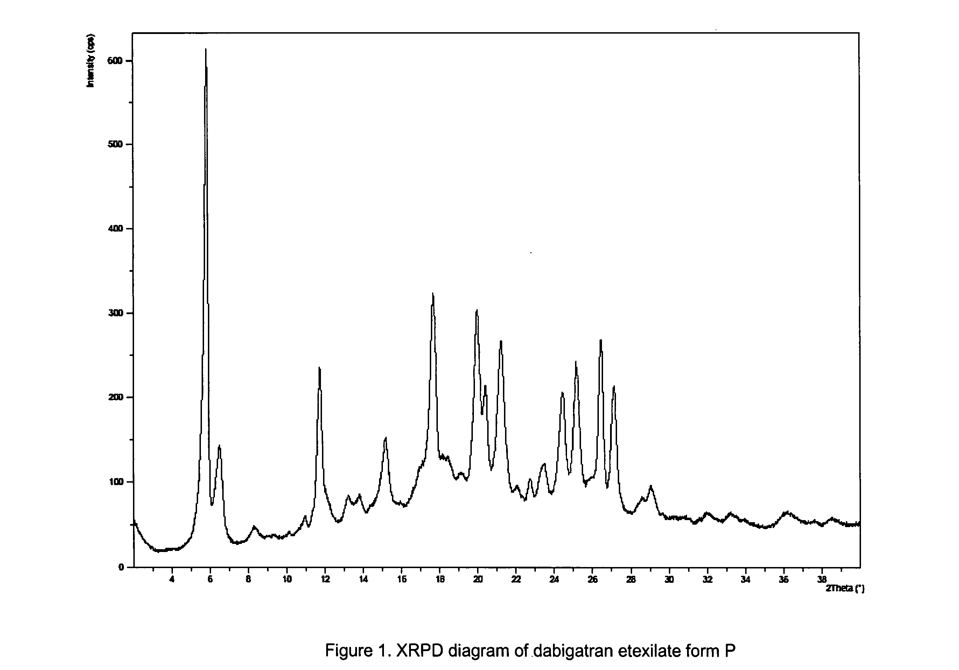

The novel polymorphic form P according to the present invention was investigated by X-ray powder diffraction. Figure 1. shows the obtained diagram and Table 1. contains the data obtained by the analysis. Form P is characterized by the following¹H NMR and ¹³C NMR data:
δ ¹H NMR:
   0.86 (t, 3H), 1.04 (d, 3,48H), 1.12 (t, 3 H), 1.30 (m, 6H), 1.58 (m, 2H), 2.68 (t, 2H), 3.77 (s, 3 H), 3.97 (m, 4H), 4,23 (t, 2H), 4.35 (m, 0,65H), 4.59 (d, 2H), 6.76 (d, 2H), 6,89 (d, 1H), 6.96 (t, 1H), 7.12 (dd, 1H), 7.16 (d, 1H), 7.40 (d, 1H), 7.47 (s, 1H), 7.54 (dt, 1H), 7.80 (d, 2H), 8.39 (d, 1H), 8,63 (bs, 1H), 9,16 (bs, 1H)
δ¹³C NMR:
   13.9 (CH3), 22.0 (CH2), 25.2 (CH2), 25.5 (i-PrOH), 28.5 (CH2), 29.9 (CH3), 31.0 (CH2), 33.0 (CH2), 39.5 (m, CH2), 44.3 (CH2), 60.0 ((CH2), 62.0 (i-PrOH), 64.1 (CH2), 109.5 (CH), 111.3 (CH), 119.5 (CH), 121.0 (CH), 121.2 (CH), 122.1 (CH), 122.8 (CH), 129.1 (CH), 129.3 (C-q), 137.2 (C-q), 137.9 (CH), 140.8 (C-q), 148,7 (CH), 151.6 (C-q), 153.7 (C-q), 156.0 (C-q), 164.2 (N-(C=O)-O), 166.4 (C-q), 170.3 (C=O), 171.0 (C=O)

**Table 1.**

| **No.** | **Pos. [°2Th.]** | **d-spacing [Å]** | **Rel. Int. [%]** |
|---|---|---|---|
| 1 | 5.8447 | 15.12168 | 100.00 |
| 2 | 6.5188 | 13.55942 | 19.50 |
| 3 | 8.3265 | 10.61923 | 2.72 |
| 4 | 9.3837 | 9.42506 | 0.64 |
| 5 | 10.1349 | 8.72806 | 0.71 |
| 6 | 10.9568 | 8.07511 | 3.01 |
| 7 | 11.7554 | 7.52828 | 31.89 |
| 8 | 13.2293 | 6.69266 | 4.69 |
| 9 | 13.8063 | 6.41427 | 4.26 |
| 10 | 15.1744 | 5.83887 | 14.12 |
| 11 | 16.9398 | 5.23415 | 6.73 |
| 12 | 17.6812 | 5.01632 | 40.81 |
| 13 | 18.4252 | 4.81541 | 8.49 |
| 14 | 19.1202 | 4.64189 | 5.06 |
| 15 | 19.9792 | 4.44423 | 37.73 |
| 16 | 20.4057 | 4.35230 | 22.44 |
| 17 | 21.2291 | 4.18531 | 31.56 |
| 18 | 22.0654 | 4.02853 | 2.57 |
| 19 | 22.7393 | 3.91064 | 4.02 |
| 20 | 23.2786 | 3.82126 | 5.59 |
| 21 | 23.4700 | 3.79052 | 7.35 |
| 22 | 24.4447 | 3.64154 | 21.77 |
| 23 | 25.1731 | 3.53781 | 27.67 |
| 24 | 26.4507 | 3.36975 | 33.93 |
| 25 | 27.1227 | 3.28777 | 24.74 |
| 26 | 28.6223 | 3.11883 | 3.32 |
| 27 | 29.0611 | 3.07274 | 5.85 |
| 28 | 30.7746 | 2.90544 | 0.73 |
| 29 | 32.0253 | 2.79478 | 2.11 |
| 30 | 33.2133 | 2.69747 | 2.38 |
| 31 | 34.0045 | 2.63650 | 1.23 |
| 32 | 36.1772 | 2.48299 | 2.94 |
| 33 | 37.5966 | 2.39245 | 1.01 |
| 34 | 38.5225 | 2.33705 | 1.32 |

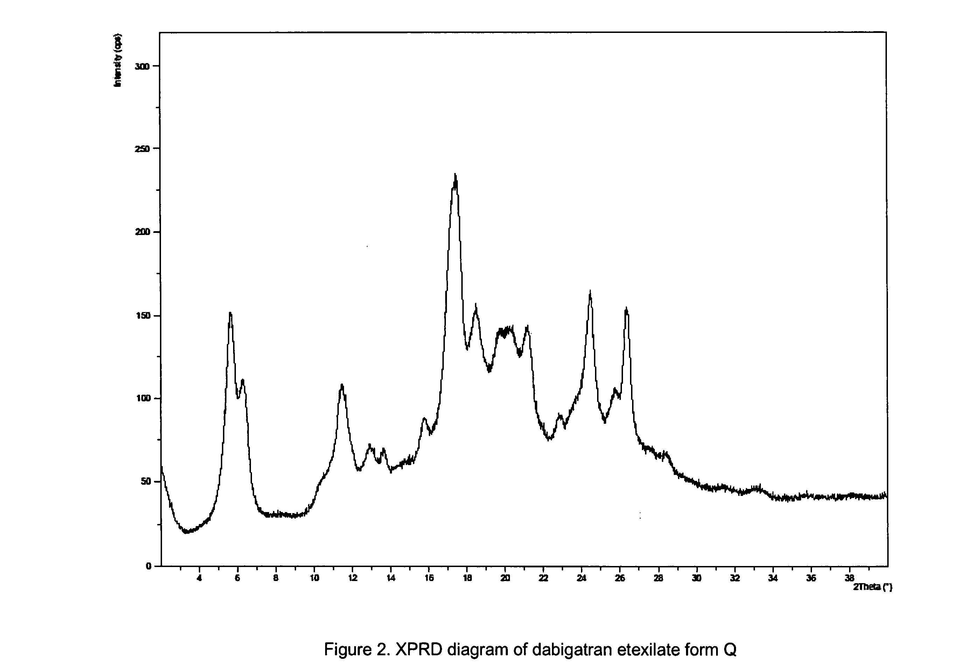

The novel polymorphic form Q according to the present invention was investigated by X-ray powder diffraction. Figure 2. shows the obtained diagram and Table 2. contains the data obtained by the analysis. Form Q is characterized by the following ¹H NMR and ¹³C NMR data:

**Table 2.**

| **No.** | **Pos. [°2Th.]** | **d-spacing [Å]** | **Rel. Int. [%]** |
|---|---|---|---|
| 1 | 5.6827 | 15.55243 | 78.82 |
| 2 | 6.2975 | 14.03535 | 53.13 |
| 3 | 10.2850 | 8.60103 | 7.98 |
| 4 | 11.4809 | 7.70764 | 40.15 |
| 5 | 12.9621 | 6.83003 | 12.11 |
| 6 | 13.7034 | 6.46220 | 7.56 |
| 7 | 15.7951 | 5.61080 | 14.11 |
| 8 | 17.4419 | 5.08459 | 100.00 |
| 9 | 18.4880 | 4.79918 | 49.83 |
| 10 | 19.7698 | 4.49083 | 40.53 |
| 11 | 20.2641 | 4.38238 | 41.43 |
| 12 | 21.1611 | 4.19859 | 41.56 |
| 13 | 22.8733 | 3.88804 | 10.04 |
| 14 | 24.4636 | 3.63878 | 57.97 |
| 15 | 26.3845 | 3.37805 | 56.27 |
| 16 | 27.5624 | 3.23631 | 6.86 |
| 17 | 28.3965 | 3.14312 | 7.20 |
| 18 | 31.4153 | 2.84764 | 2.17 |
| 19 | 33.1557 | 2.70203 | 2.81 |
| 20 | 35.7646 | 2.51069 | 1.04 |
| 21 | 38.0825 | 2.36303 | 0.77 |

The present invention further relates to processes for producing the novel polymorphic forms of ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate. The starting material, ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate can be obtained by methods known from the prior art, e.g. according to WO98/037075, Example 113 as an oily product. According to the invention the form P of dabigatran etexilate is obtained by
a) dissolving ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate in isopropanol
b) distilling out half of the solvent and adding fresh solvent
c) cooling to ambient temperature, filtering the product, washing with isopropanol and drying.

In another aspect of the invention the form P is obtained by
a) dissolving ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate in a mixture of toluene/ethanol and separating the phases
b) concentrating the toluene phase under vacuum and dissolving the residue in isopropanol
c) keeping the mixture at ambient temperature
d) filtering the product, washing with isopropanol and drying.

In a further aspect of the invention the form P is obtained by
a) mixing amorphous ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate with isopropanol
b) stirring the mixture at ambient temperature
c) filtering the product, washing with isopropanol and drying.

In a further aspect of the invention the form Q is obtained by
a) suspending ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate in isopropanol and heating it until dissolution is complete
b) cooling the solution and maintaining it at 55 - 50 °C
c) cooling the mixture to ambient temperature and stirring it
d) filtering the product, washing with isopropanol and drying.

### Examples

The present invention is illustrated by the following examples which limit in no way its scope.

### Example 1

250 g of the oily residue prepared as described in WO98/037075 is dissolved in a mixture of toluene/ethanol. The formed phases are separated. The toluene phase was concentrated under vacuum and the residue (purity by HPLC 96%) is dissolved in isopropanol. The mixture is kept at ambient temperature within three hours. The solid formed is filtered off, washed with isopropanol and dried at 40°C.

Yield: 130 g; purity by HPLC 99,8 %; GC/HS: isopropanol 6,7 %.

### Example 2

Amorphous ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate 6,1 g (HPLC 97,6 %) is mixed with 65 ml isopropanol and stirred for two hours at ambient temperature. Then the product is filtered, washed with isopropanol and dried. Yield: 5,8 g; (HPLC 98,7%).

### Example 3

Crude ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate in amount 29,5 g (89 %, HPLC) prepared as described in example 1 is suspended in 200 ml isopropanol and heated to obtain solution. 110 ml of the solvent is subsequently distilled out and fresh isopropanol in amount 110 ml is added. The mixture is cooled to ambient temperature and stirred. Then the product is filtered, washed with isopropanol and dried. Yield: 12,8 g; (99,2 %, HPLC).

### Example 4

30 g of ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate is suspended in 240 ml isopropanol and heated to about 65°C until dissolution is complete. The mixture is cooled to 55°C and maintained at 55 - 50 °C for 2 h. Then it is cooled to ambient temperature and stirring for a few hours. The product is filtered off, washed with isopropanol and dried. Yield: 27 g;

### Example 5

Tablet. API is 50 mg of ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate form P

Composition:

| | |
|---|---|
| a) API | 50.0 |
| b) Lactose | 100.0 |
| c) Starch | 50.0 |
| d) Hydroxypropylcellulose | 13.0 |
| e) Magnesium stearate | 2.0 |

Preparation: API, lactose and starch were mixed. The mixture was granulated with the aqueous-ethanolic solution of hydroxypropylcellulose. Magnesium stearate was added to the dried granulation and mixed. Tablets were pressed from this mixture.

### Example 6

Tablet. API is 50 mg of ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate form Q

Composition:

| | |
|---|---|
| a) API | 50.0 |
| b) Lactose | 100.0 |
| c) Starch | 50.0 |
| d) Hydroxypropylcellulose | 13.0 |
| e) Magnesium stearate | 2.0 |

Preparation: API, lactose and starch were mixed. The mixture was granulated with the aqueous-ethanolic solution of hydroxypropylcellulose. Magnesium stearate was added to the dried granulation and mixed. Tablets were pressed from this mixture

## Claims

1. Ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanonate in crystalline form (form P) having X-ray powder diffractogram with the characteristic peaks shown in Figure 1.

2. The compound according to claim 1, wherein the reflections at about 5.9, 6.5, 11.8, 15.2, 17.7, 20.0, 21.2, 24.5, 25.2, 26.5 and 27.1 ± 2°θ.

3. Ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanonate in crystalline form (form Q) having X-ray powder diffractogram with the characteristic peaks shown in Figure 2.

4. The compound according to claim 3, wherein the reflections at about 5.7, 6.3, 11.5, 17.4, 18.5, 21.2, 24.5, 26.4 ± 2°θ.

5. Process for preparing ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanonate according to claims 1-2, **characterized in that**
a) mixing amorphous ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate with isopropanol
b) stirring the mixture at ambient temperature
c) filtering the product, washing with isopropanol and drying.

6. Process for preparing ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanonate according to claims 1-2, **characterized in that**
a) dissolving ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate in isopropanol
b) distilling out half of the solvent and adding fresh solvent
c) cooling to ambient temperature, filtering the product, washing with isopropanol and drying.

7. Process for preparing ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanonate according to claims 1-2, **characterized in that**
a) dissolving ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate in a mixture of toluene/ethanol and separating the phases
b) concentrating the toluene phase under vacuum and dissolving the residue in isopropanol
c) keeping the mixture at ambient temperature
d) filtering the product, washing with isopropanol and drying.

8. Process for preparing ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanonate according to claims 3-4, **characterized in that**
a) suspending ethyl 3-[N-(2-{(4-(hexyloxycarbonylamino-imino-methyl)-phenylamino]-methyl}-1-methyl-1H-benzimidazole-5-carbonyl)-N-pyridin-2-yl-amino]-propanoate in isopropanol and heating it until dissolution is complete
b) cooling the solution and maintaining it at 55 - 50 °C
c) cooling the mixture to ambient temperature and stirring it
d) filtering the product, washing with isopropanol and drying.

9. Use the compound according to claims 1-4 for preparing a pharmaceutical
composition with the effect of prolonging the thrombin time.

10. Use the compound according to claims 1-4 for preparing a pharmaceutical
composition for the prevention of venous thrombosis and stroke.

11. Pharmaceutical composition consisting of a compound of claims 1-4 optionally together with one or more excipients.
